# EUROPEAN PATENT SPECIFICATION

(11) **EP 3 847 155 B1**
(45) Date of publication and mention of the grant of the patent: **02.09.2026**
(21) Application number: 19782721.5
(22) Date of filing: 02.09.2019
(51) Int. Cl.: C07C 253/20, C07C 255/10, C07D 257/04

(54) **A METHOD FOR THE PREPARATION OF FLUOROALKYL NITRILES AND THEIR USE TO PREPARE RELATED FLUOROALKYL TETRAZOLES**
VERFAHREN ZUR HERSTELLUNG VON FLUORALKYLNITRILEN UND DEREN VERWENDUNG ZUR HERSTELLUNG VON ENTSPRECHENDEN FLUORALKYLTETRAZOLEN
PROCÉDÉ DE PRÉPARATION DE FLUOROALKYL NITRILES ET LEUR UTILISATION DANS LA PRÉPARATION DE FLUOROALKYL TÉTRAZOLES ASSOCIÉS

(30) Priority: 03.09.2018 IN 201811033029
(43) Date of publication of application: 14.07.2021
(73) Proprietor: PI Industries Ltd., Udaipur, Rajasthan 313001 (IN)
(72) Inventor: KARRI, Phaneendrasai, Guntur-Andhra Pradesh 522006 (IN); PABBA, Jagadish, Udaipur- Rajasthan 313001 (IN); GURJAR, Bhagwan Lal, Udaipur-Rajasthan 313002 (IN); KLAUSENER, Alexander G.M., 50259 Pulheim (DE)
(74) Representative: Keltie LLP
(86) International application number: PCT/IB2019/057377
(87) International publication number: WO 2020/049436

(56) References cited:
- WO-A1-2018/019693
- CN-A- 102 746 190
- CN-A- 103 804 231
- CRAWFORD M J ET AL: "Synthesis and characterization of perfluorinated nitriles and the corresponding sodium 5-perfluoroalkyltetrazolate salts", JOURNAL OF FLUORINE CHEMISTRY, ELSEVIER, NL, vol. 129, no. 12, 1 December 2008 (2008-12-01), pages 1199 - 1205, XP025678338, ISSN: 0022-1139, [retrieved on 20080925], DOI: 10.1016/J.JFLUCHEM.2008.09.007

## Description

### FIELD OF THE INVENTION:

The present invention relates to a method for the preparation of fluoroalkyl nitriles and their use to prepare related fluoroalkyl tetrazoles.

### BACKGROUND:

Fluoroalkyl nitriles and the fluoroalkyl tetrazoles prepared from them are important precursors and are widely used in chemical and pharmaceutical industry. For example, fluoroalkyl nitriles are used as starting material or intermediate in preparing polymer and agrochemical products, whereas fluoroalkyl tetrazoles are used as intermediates in preparing pharmaceutical and agrochemical active ingredients.

Due to its widespread utilities there are several efforts being made in the direction to provide a simple, economic, high yielding and commercially amenable process for preparing fluoroalkyl nitriles and for their use to prepare fluoroalkyl tetrazoles.

For instance, Swarts et al., in Bulletin Societes Chimiques Beiges, 1922, Vol 31, 364-365; Jones et al., in Journal of Organic Chemistry 1943, 65, 1458; and Grunewald et al., in J. Med. Chem. 2006, 49, 2939-2952; disclose processes wherein phosphorus pentoxide is used as a dehydrating agent for dehydrating fluoroalkyl amides into fluoroalkyl nitriles.

CN102746190 discloses a process for the preparation of fluoroalkyl nitriles from fluoroalkyl amides using phosphorus pentoxide as a dehydrating agent and phosphoric acid or polyphosphoric acid as a solvent.

CN103804231 discloses a process for the preparation of trifluoromethyl nitrile from trifluoromethyl amide using triphenyl phosphine, trifluoroacetic anhydride and pyridine in carbon tetrachloride as a solvent. CN103804231 emphasizes the use of carbon tetrachloride as a solvent and triphenyl phosphine as a reagent which during the reaction forms an intermediate CF₃C(Cl)=NH. The intermediate upon further reaction with the second molecule of triphenyl phosphine gives trifluoromethyl nitrile.

These processes involving the use of phosphorus containing reagent suffer from one or the other disadvantage. For examples, these processes require special reactors, are conducted at higher temperature conditions, and are not commercially amenable due to the use of expensive and dangerous reagents.

US2010312002 describes the preparation of fluoroalkyl nitriles from fluoroalkyl amides using acid halides as a dehydrating reagent. The problem associated with the use of acid halides as a dehydrating reagent is that hydrogen halide is generated as a by-product. Therefore, the fluoroalkyl nitriles prepared using this process cannot be used as reactants to react with sodium azide to form the corresponding fluroalkyl tetrazoles for the reason that hydrogen halide and sodium azide together react to form unstable and extremely explosive hydrazoic acid (HN₃).

Parker et al., in Synthetic Communications, Volume 34, 2004, 903-907 and Crawford M J et al., in Journal of Fluorine Chemistry, 129, 2008, 1199-1205 describe the preparation of trifluoromethyl nitrile by dehydrating trifluoromethyl amide using trifluoroacetic anhydride as a dehydrating reagent in the presence of a base such as pyridine.

WO2018019693 discloses a process for the preparation of fluoroalkyl nitriles in the presence of a base (such as pyridine, picolines, quinoline, quinalidine and halogenated pyridines) and phosphorus trichloride and/or phosphorus oxychloride as reagents.

The disadvantage associated with the processes described by Parker et al., and Crawford et al., is that pyridinium trifluoroacetate salt is formed as a by-product. The recovery of pyridine and trifluoro acetic acid and converting trifluoro acetic acid back into trifluoro acetic anhydride adds to the cost of operation and production. Another disadvantage of these two processes is that the trifluoro acetic acid generated during the reaction reacts exothermically with the base present in the medium leading to a rise in temperature. The rise in the temperature may cause uncontrolled generation of trifluoromethyl nitrile which, if not consumed at real time, may have to be condensed at -196 °C which requires huge operational cost.

Norris et al., in Journal of Organic Chemistry, 1962, 27, 1449, discloses a process for preparing trifluoromethyl tetrazole by reacting sodium azide and trifluoromethyl nitrile in acetonitrile at a temperature of 60 °C.

Thus, there is still an unmet need for a simple, economic, high yielding and commercially amenable process for preparing fluoroalkyl nitriles and the corresponding fluoroalkyl tetrazoles derived from them.

Accordingly, the present invention provides a method for the preparation of fluoroalkyl nitriles and the corresponding fluoroalkyl tetrazoles derived from them which obviate at least one of the above listed disadvantages.

### OBJECT AND SUMMARY OF THE INVENTION:

It is therefore an object of the present invention to provide a simple, safe, economical, and commercially amenable method for the preparation of fluoroalkyl nitrile of Formula I and for their use to prepare the corresponding fluoroalkyl tetrazoles of Formula II.

Another object of the present invention is to provide a high yielding method for the preparation of fluoroalkyl nitrile of Formula I and for their use to prepare the corresponding fluoroalkyl tetrazoles of Formula II having high purity.

These objects were achieved according to the present invention by providing a method for the preparation of fluoroalkyl nitrile of Formula I; 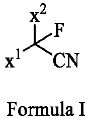
wherein, x¹ and x² are fluorine,
by dehydrating a fluoroalkyl amide of Formula III; 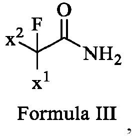
in the absence of a base, using one or more dehydrating agent(s) optionally in the presence of one or more suitable solvent(s), wherein the dehydrating agent is selected from the group consisting of acetic anhydride, difluoroacetic anhydride, trifluroacetic anhydride, trifluoromethanesulfonic anhydride, p-toluenesulfonic acid, methanesulfonic acid, trifluoromethanesulfonic acid, acetic acid, difluoroacetic acid, and trifluroacetic acid.

Fluoroalkyl nitrile of Formula I are neutralized by interacting with one or more base(s) selected from alkylamine, dialkylamine, trialkylamine, pyridine, halogenated pyridine, 3-picoline, 4-picoline, quinoline, quinaldine, and alkylpyridine to remove any trapped traces of acid, generated during the course of the dehydration reaction, before reacting it with sodium azide in the presence of one or more suitable solvent(s) to obtain the corresponding fluoroalkyl tetrazole of Formula II; 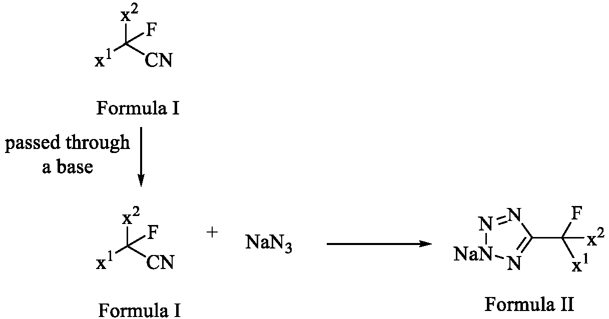 wherein, x¹ and x² are as defined in the description.

In the present invention, fluoroalkyl nitrile of Formula I is generated using only a dehydrating agent, optionally with simultaneous removal of the by-product obtained from the dehydrating agent during and or after the dehydration reaction. Unlike in the prior art, due to the absence of the base during the dehydration reaction, recycling of acids such as for example trifluoroacetic acid generated during the course of the dehydration reaction is very easy and inexpensive.

Further, fluoroalkyl nitrile of Formula I is passed through a base before reacting with sodium azide thereby preventing the formation of hydrazoic acid (HN₃). This makes the present process safe.

### DETAILED DESCRIPTION OF THE INVENTION:

The present invention relates to a method for the preparation of fluoroalkyl nitrile of Formula I; 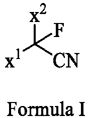
wherein, x¹ and x² are fluorine,
by dehydrating a fluoroalkyl amide of Formula III; 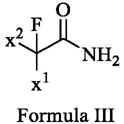
wherein, x¹ and x² have the same meaning as defined herein above,
in the absence of a base, using one or more dehydrating agent(s) optionally in the presence of one or more suitable solvent(s),
wherein the one or more dehydrating agent(s) are selected from the group consisting of acetic anhydride, difluoroacetic anhydride, trifluroacetic anhydride, trifluoromethanesulfonic anhydride, p-toluenesulfonic acid, methanesulfonic acid, trifluoromethanesulfonic acid, acetic acid, difluoroacetic acid, and trifluroacetic acid. ,

The preparation of fluoroalkyl nitrile of Formula I may be carried out in the absence of a solvent or in the presence of one or more suitable solvent(s).

The temperature conditions required for the preparation of fluoroalkyl nitrile of Formula I according to the present invention may vary depending on the starting materials used which may range from -40 °C to 250 °C, particularly in the range from -15 °C to 180 °C, more particularly in the range from -4 °C to 120 °C and most particularly in the range from 5 °C to 70 °C.

The dehydration reaction for the preparation of fluoroalkyl nitrile of Formula I can generally be carried out under reduced pressure or at atmospheric pressure or excess pressure.

In one embodiment, the respective fluoroalkyl amide of Formula III is added to the dehydrating agent.

In another embodiment, the dehydrating agent is added to the respective fluoroalkyl amide of Formula III.

In yet another embodiment, a mixture of the respective fluoroalkyl amide of Formula III and one or more suitable solvent(s) are added to the dehydrating agent.

In yet another embodiment, a mixture of the dehydrating agent and one or more suitable solvent(s) is added to the respective fluoroalkyl amide of Formula III.

In yet another embodiment, the dehydrating agent is added to a mixture of the respective fluoroalkyl amide of Formula III and one or more suitable solvent(s).

In yet another embodiment, the respective fluoroalkyl amide of Formula III is added to the mixture of the dehydrating agent and one or more solvent(s).

In yet another embodiment, a mixture of the respective fluoroalkyl amide of Formula III and one or more first suitable solvent(s) is added to a mixture of the dehydrating agent and one or more second suitable solvent(s). The first suitable solvent and the second suitable solvent may be the same or different.

In yet another embodiment, a mixture of the dehydrating agent and one or more first suitable solvent(s) is added to a mixture of the respective fluoroalkyl amide of Formula III and one or more second suitable solvent(s). The first suitable solvent(s) and the second suitable solvent(s) may be the same or different.

The amount of the dehydrating agent should be sufficient to allow substantial or complete dehydration of fluoroalkyl amide of Formula III. The molar ratio of the dehydrating agent to fluoroalkyl amide of Formula III can be between 0.5:1 and 50:1, particularly between 0.5:1 and 20:1, more particularly between 1:1 and 10:1, most particularly between 1:1 and 5:1.

The concentration of fluoroalkyl amide of Formula III in a solution containing solvent and fluoroalkyl amide of Formula III may vary from 1.0 w/w% to 99.0 w/w%.

The concentration of the dehydrating agent in a solution containing one or more solvent(s) and the dehydrating agent may vary from 1.0 w/w% to 99.0 w/w%.

In one embodiment of the present invention, the by-product obtained from the dehydrating agent during and or after the dehydration reaction is not removed.

In another embodiment of the present invention, the by-product obtained from the dehydrating agent during and or after the dehydration reaction is simultaneously removed.

The respective fluoroalkyl nitrile of Formula I prepared by the method described hereinabove is interacted with one or more base(s) selected from alkylamine, dialkylamine, trialkylamine, pyridine, halogenated pyridine, 3-picoline, 4-picoline, quinoline, quinaldine, or alkylpyridine; and subsequently reacted with sodium azide in the presence of one or more suitable solvent(s) to obtain fluoroakyl tetrazole of Formula II; 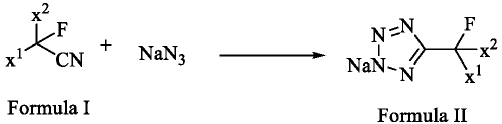 wherein, x¹ and x² are as defined hereinabove.

The interaction of fluoroalkyl nitrile of Formula I with a base can be carried out by way of bubbling, purging, frizzing, dripping or by any other means that brings fluoroalkyl nitrile of Formula I prepared according to the method of the present invention in physical contact with a base.

In one embodiment, fluoroalkyl nitrile of Formula I prepared according to the method of the present invention can be passed through a base or a mixture of bases.

In another embodiment, fluoroalkyl nitrile of Formula I prepared according to the method of the present invention can be passed through series of bases wherein bases in the series may be same or different.

In yet another embodiment, fluoroalkyl nitrile of Formula I prepared according to the method of the present invention can be passed through series of bases wherein each individual component of the series can be a mixture of bases.

The base through which fluoroalkyl nitrile of Formula I prepared according to the method of the present invention can be passed include alkylamines such as methylamine, ethylamine, propylamine, isopropylamine, n-butylamine, sec-butylamine, tert-butylamine, isobutylamine, pentylamine, and hexylamine; dialkylamines such as dimethylamine, diethylamine, ethylmethylamine, dipropylamine, diisopropylamine, dibutylamine, and methylhexanamine; trialkylamines such as *N, N-*diisopropylethylamine, 1,3-dimethylbutylamine, *N*, *N*-dimethylethylamine, tributylamine, triethylamine, triisopropylamine, trimethylamine; pyridine, halogenated pyridines such as 2- 3- or 4- chloro pyridine, 2, 6-dichloropyridine, 2, 4-dichloropyridine, 2, 4, 6-trichloropyridine, 3-picoline, 4-picoline, quinoline, quinaldine, and alkylpyridines for example 2,6-dimethylpyridine, 2-methyl-5-ethylpyridine, and 2,3-dimethylpyridine, but are not limited to these examples.

The temperature conditions required for the preparation of fluoroalkyl tetrazole of Formula II according to the present invention may vary depending on the starting materials used which may range from -80 °C to 250 °C, particularly in the range from -10 °C to 180 °C, more particularly in the range from 0 °C to 120 °Cand most particularly in the range from 10 °C to 70 °C.

The reaction of sodium azide and fluoroalkyl nitrile of Formula I can generally be carried out under reduced pressure or at atmospheric pressure or excess pressure.

The amount of sodium azide should be sufficient to consume the generated fluoroalkyl nitrile of Formula I. The molar ratio of sodium azide to fluoroalkyl nitrile of Formula I can be in between 1:1 and 1:10, particularly between 1:1 and 1:5, more particularly between 1:1 and 1:2.

Examples of solvents suitable for the preparation of fluoroalkyl nitrile of Formula I and their use to prepare related fluoroalkyl tetrazole of Formula II include acetonitrile, carbon tetrachloride, chloroform, dichloromethane, acetone, γ-butyrolactone, N-methyl-2-pyrrolidone, nitromethane, dimethylformamide, tetramethyl urea, dimethylpropylene urea, dimethylsulfoxide, sulfolane, dimethyl carbonate, ethylene carbonate, trifluoroacetic acid, difluoroacetic acid, and acetic acid but are not limited to these examples, particularly, acetone or acetonitrile, but are not limited to these examples.

The concentration of sodium azide in a solution containing one or more solvent(s) and sodium azide may vary from 1.0 w/w% to 99.0 w/w%.

The fluoroalkyl amide of Formula III used in the present invention are either commercially available or can be easily prepared by processes known from the literature WO2003080563; Swarts et al., Bulletin Societes Chimiques Beiges, 1922, Vol 31, 364-365; J. King, 1961, 27-28, thesis titled "A study of the synthesis of 2-trifluoromethyl primidines".

Also disclosed (but not claimed) herein is an apparatus for the preparation of fluoroalkyl tetrazole of Formula II, said apparatus comprising:
a. a first reactor equipped with means for addition, in which the dehydration of fluoroalkyl amide of Formula III giving fluoroalkyl nitrile of Formula I in the presence of the dehydrating agent is carried out,
b. a vessel or a plurality of vessels in a series or a scrubber comprising one or more base(s) with means for accepting and passing fluoroalkyl nitrile of Formula I through a base to purify fluoroalkyl nitrile of Formula I, and
c. a second reactor equipped with means for accepting the purified fluoroalkyl nitrile of Formula I, in which sodium azide and the purified fluoroalkyl nitrile of Formula I react to obtain fluoroalkyl tetrazole of Formula II.

The first reactor may be a flow reactor, wherein the dehydration of the respective fluoroalkyl amide of Formula III using one ore more dehydrating agents, optionally in the presence of one or more suitable solvent(s) is carried out during the flow of reacting components and solvent, if used, at a temperature ranging -40 °C to 180 °C, particularly in the range from -15 °C to 150 °C, more particularly in the range from -4 °C to 100 °C and most particularly in the range from 5 °C to 60 °C.

The reacted material after leaving the flow reactor may then be separated in a conventional way by condensing unreacted material as the respective unreacted fluoroalky amide, unreacted dehydrating agent, reaction product which has formed from the dehydrating agent and water, as well as other condensable side products, and by this means separating it from the gaseous respective fluoroalkyl nitrile which is used, preferably after a further purification step as the above mentioned contacting with an appropriate base to remove any potential acid traces, in order to use it for the preparation of the respective fluoroalkyl tetrazole.

The first reactor may be a column reactor having three zones, a first zone, a second zone and a third zone.

The first zone can be operated at a temperature suitable to separate the dehydrating agent and the product formed from the dehydrating agent during the dehydration reaction. Thus, the temperature in the first zone may vary from 25 °C to 180 °C depending upon the boiling or melting point difference between the dehydrating agent and that of the product formed therefrom after the dehydration reaction.

The second zone can be operated at a temperature suitable to cause the dehydration reaction of fluoroalkyl amide of Formula III and simultaneously obviate interference of the by-product formed from the dehydrating agent during the dehydration reaction. Suitably, the temperature in the second zone may vary from 30 °C to 175 °C.

The third zone is operated at a temperature suitable to condense the fluoroalkyl amide of Formula III, the dehydrating agent and the solvent(s), if used but not to condense fluoroalkyl nitrile of Formula I generated in the second zone of the column reactor.

The by-product generated during the dehydration reaction in the first reactor, for example, trifluoroacetic acid in case the dehydrating agent used is trifluoroacetic anhydride, along with the solvent(s), if used, is collected in a separate vessel for recycling. The Fluoroalkyl nitrile of Formula I prepared in the first reactor is then passed through a vessel or a plurality of vessels in a series or a scrubber comprising one or more base(s) before reacting with sodium azide in the second reactor.

The invention is illustrated in further details with the help of the following examples, without imposing any limitation thereto.

### CHEMISTRY EXAMPLES:

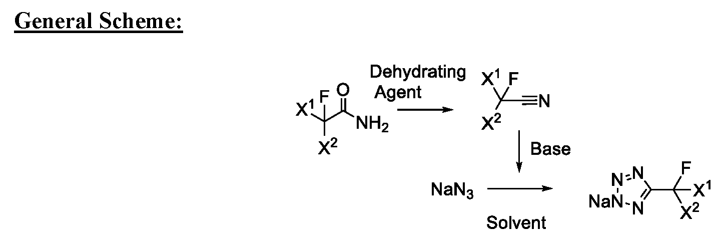

### Example 1:

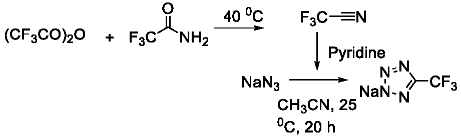

A solution of trifluoromethyl amide (5.6 g, 50 mmol) and trifluoroacetic acid (3 mL) was slowly added to 2,2,2-trifluoroacetic anhydride (42.2 g, 200 mmol) at 40 °C. The gaseous trifluoromethyl nitrile formed in the course of addition was passed through a trap containing pyridine (10 mL) to remove traces of trifluoroacetic acid, if any. The purified trifluoromethyl nitrile was then bubbled into a solution of sodium azide (6.5 g, 100 mmol) and acetonitrile (50 mL) at 25 °C for 15 h under efficient stirring. After completion of the reaction, the resultant suspension was filtered and the filtrate was concentrated to obtain 1.2 g (Yield: 29%) of sodium 5-(trifluoromethyl)tetrazol-1-ide.
¹⁹F-NMR (376 MHz, DMSO-D6) δ -59.48 (3F)
¹³C-NMR (101 MHz, DMSO-D6) δ 153.96-152.95 (1C), 126.74-118.10 (1C).

### Example 2:

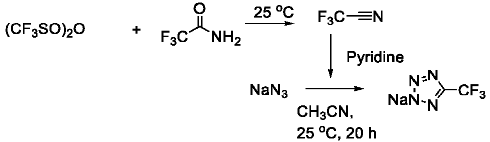

Trifluoromethanesulfonic anhydride (17 gm, 60 mmol) was added to 2,2,2-trifluoroacetamide (5.6 g, 50 mmol) at 25 °C. The gaseous trifluoroacetonitrile formed in the course of addition was passed through a trap containing pyridine (10 mL) to remove traces of trifluoromethanesulfonic acid, if any. The purified trifluoromethyl nitrile was then bubbled into a solution of sodium azide (3.25 g, 50 mmol) in acetonitrile (50 mL) at 25 °C for 20 h. The resultant suspension was filtered and the filtrate was concentrated to obtain to obtain 3.2 g (Yield: 40%) of sodium 5-(trifluoromethyl)tetrazol-1-ide.
¹⁹F-NMR (376 MHz, DMSO-D6) δ -59.48 (3F)
¹³C-NMR (101 MHz, DMSO-D6) δ 153.96-152.95 (1C), 126.74-118.10 (1C).

## Claims

1. A method for the preparation of a fluoroalkyl tetrazole of Formula II; 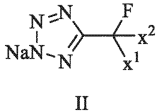
wherein, x¹ and x² are fluorine;
said method comprising the steps of:
a) dehydrating a fluoroalkyl amide of Formula III, in the absence of a base, using one or more dehydrating agent(s) optionally in the presence of one or more suitable solvent(s) to obtain a fluoroalkyl nitrile of Formula I, 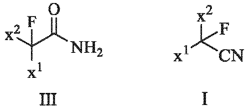 wherein, x¹ and x² are each as defined herein above;
wherein the dehydrating agent is selected from the group consisting of acetic anhydride, difluoroacetic anhydride, trifluoroacetic anhydride, trifluoromethanesulfonic anhydride, p-toluenesulfonic acid, methanesulfonic acid, trifluoromethanesulfonic acid, acetic acid, difluoroacetic acid, and trifluoroacetic acid;
b) neutralizing the fluoroalkyl nitrile of Formula I by interacting with one or more base(s) selected from alkylamine, dialkylamine, trialkylamine, pyridine, halogenated pyridine, 3-picoline, 4-picoline, quinoline, quinaldine, and alkylpyridine; and
c) reacting the neutralized fluoroalkyl nitrile of Formula I with sodium azide in the presence of one or more suitable solvent(s) to obtain the fluoroalkyl tetrazole of Formula II.

2. The method as claimed in claim 1, wherein the by-product formed during or after the dehydration reaction from the dehydrating agent is simultaneously removed.

3. The method as claimed in claim 1, wherein
a) the step (a) is carried out at a temperature ranging from -4 °C to 120 °C;
b) the solvent useful in step (a) is selected from the group consisting of acetonitrile, carbon tetrachloride, chloroform, dichloromethane, acetone, γ-butyrolactone, N-methyl-2-pyrrolidone, nitromethane, dimethylformamide, tetramethyl urea, dimethylpropylene urea, dimethylsulfoxide, sulfolane, dimethyl carbonate, ethylene carbonate, trifluoroacetic acid, difluoroacetic acid, and acetic acid;
c) the molar ratio of the dehydrating agent to the fluoroalkyl amide of Formula III ranges from 0.5:1 to 20:1;
d) the concentration of fluoroalkyl amide of Formula III in a solution containing one or more solvent(s) and fluoroalkyl amide of Formula III ranges from 1.0 w/w% to 99.0 w/w%; and
e) the concentration of the dehydrating agent in a solution containing one or more solvent(s) and the dehydrating agent ranges from 1.0 w/w% to 99.0 w/w%.

4. The method as claimed in claim 1, wherein
a) the step (b) is carried out till the pH in the range from 6.5 to 8.5 is attained; and
b) the step (b) is carried out a temperature ranging from 15 °C to 60 °C.

5. The method as claimed in claim 1, wherein
a) the solvent used in step (c) is selected from the group consisting of acetonitrile, carbon tetrachloride, chloroform, dichloromethane, acetone, γ-butyrolactone, N-methyl-2-pyrrolidone, nitromethane, dimethylformamide, tetramethyl urea, dimethylpropylene urea, dimethylsulfoxide, sulfolane, dimethyl carbonate, ethylene carbonate, trifluoroacetic acid, difluoroacetic acid, and acetic acid;
b) the step (c) is carried out a temperature ranging from 0 °C to 120 °C; and
c) the molar ratio of sodium azide to fluoroalkyl nitrile of Formula I ranges from 1:1 to 1:10.

## Patentansprüche

1. Verfahren zur Herstellung eines Fluoralkyltetrazols mit der Formel II: 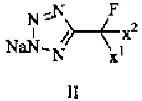
wobei x¹ und x² Fluor sind;
wobei das Verfahren die Schritte umfasst:
a) Dehydratisieren eines Fluoralkylamids der Formel III in Abwesenheit einer Base unter Verwendung eines oder mehrerer Dehydratisierungsmittel, gegebenenfalls in Gegenwart eines oder mehrerer geeigneter Lösungsmittel, um ein Fluoralkylnitril der Formel I zu erhalten, 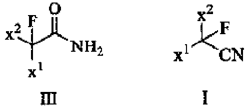
wobei x¹ und x² jeweils wie zuvor definiert sind;
wobei das Dehydratisierungsmittel ausgewählt ist aus der Gruppe bestehend aus Essigsäureanhydrid, Difluoressigsäureanhydrid, Trifluoressigsäureanhydrid, Trifluormethansulfonsäureanhydrid, p-Toluolsulfonsäure, Methansulfonsäure, Trifluormethansulfonsäure, Essigsäure, Difluoressigsäure und Trifluoressigsäure;
b) Neutralisieren des Fluoralkylnitrils der Formel I durch Interagieren mit einer oder mehreren Base(n) ausgewählt aus Alkylamin, Dialkylamin, Trialkylamin, Pyridin, halogeniertem Pyridin, 3-Picolin, 4-Picolin, Chinolin, Chinaldin und Alkylpyridin; und
c) Umsetzen des neutralisierten Fluoralkylnitrils der Formel I mit Natriumazid in Gegenwart von einem oder mehreren geeigneten Lösungsmittel(n), um das Fluoralkyltetrazol mit Formel II zu erhalten.

2. Verfahren nach Anspruch 1, wobei das während oder nach der Dehydratisierungsreaktion aus dem Dehydratisierungsmittel gebildete Nebenprodukt gleichzeitig entfernt wird.

3. Verfahren nach Anspruch 1, wobei
a) der Schritt (a) bei einer Temperatur im Bereich von -4 °C bis 120 °C durchgeführt wird;
b) das in Schritt (a) brauchbare Lösungsmittel ausgewählt ist aus der Gruppe bestehend aus Acetonitril, Kohlenstofftetrachlorid, Chloroform, Dichlormethan, Aceton, γ-Butyrolacton, N-Methyl-2-pyrrolidon, Nitromethan, Dimethylformamid, Tetramethylharnstoff, Dimethylpropylenharnstoff, Dimethylsulfoxid, Sulfolan, Dimethylcarbonat, Ethylencarbonat, Trifluoressigsäure, Difluoressigsäure und Essigsäure;
c) das Molverhältnis des Dehydratisierungsmittels zu dem Fluoralkylamid der Formel III im Bereich von 0,5:1 bis 20:1 liegt;
d) die Konzentration des Fluoralkylamids der Formel III in einer Lösung, die ein oder mehrere Lösungsmittel und Fluoralkylamid der Formel III enthält, im Bereich von 1,0 % (Gew./Gew.) bis 99,0 % (Gew./Gew.) liegt; und
e) die Konzentration des Dehydratisierungsmittels in einer Lösung, die ein oder mehrere Lösungsmittel und das Dehydratisierungsmittel enthält, im Bereich von 1,0 % (Gew./Gew.) bis 99,0 % (Gew./Gew.) liegt.

4. Verfahren nach Anspruch 1, wobei
a) der Schritt (b) durchgeführt wird, bis der pH-Wert im Bereich von 6,5 bis 8,5 erreicht wird; und
b) der Schritt (b) bei einer Temperatur im Bereich von 15 °C bis 60 °C durchgeführt wird.

5. Verfahren nach Anspruch 1, wobei
a) das in Schritt (c) verwendete Lösungsmittel ausgewählt ist aus der Gruppe bestehend aus Acetonitril, Kohlenstofftetrachlorid, Chloroform, Dichlormethan, Aceton, γ-Butyrolacton, N-Methyl-2-pyrrolidon, Nitromethan, Dimethylformamid, Tetramethylharnstoff, Dimethylpropylenharnstoff, Dimethylsulfoxid, Sulfolan, Dimethylcarbonat, Ethylencarbonat, Trifluoressigsäure, Difluoressigsäure und Essigsäure;
b) der Schritt (c) bei einer Temperatur im Bereich von 0 °C bis 120 °C durchgeführt wird; und
c) das Molverhältnis von Natriumazid zu Fluoralkylamid der Formel I im Bereich von 1:1 bis 1:10 liegt.

## Revendications

1. Procédé de préparation d'un fluoroalkyltétrazole de formule II : 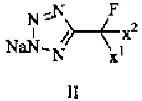
dans laquelle x¹ et x² sont le fluor ;
ledit procédé comprenant les étapes suivantes :
a) la déshydratation d'un fluoroalkylamide de formule III, en l'absence d'une base, à l'aide d'un ou plusieurs agent(s) déshydratant(s), éventuellement en présence d'un ou plusieurs solvant(s) approprié(s), afin d'obtenir un fluoroalkylnitrile de formule I, 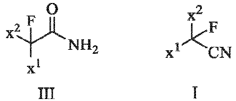
dans lesquelles x¹ et x² sont chacun tels que définis ci-dessus ;
dans lequel l'agent déshydratant est choisi dans le groupe constitué par l'anhydride acétique, l'anhydride difluoroacétique, l'anhydride trifluoroacétique, l'anhydride trifluorométhanesulfonique, l'acide p-toluènesulfonique, l'acide méthanesulfonique, l'acide trifluorométhanesulfonique, l'acide acétique, l'acide difluoroacétique et l'acide trifluoroacétique ;
b) la neutralisation du fluoroalkylnitrile de formule I par interaction avec une ou plusieurs base(s) choisie(s) parmi une alkylamine, une dialkylamine, une trialkylamine, la pyridine, une pyridine halogénée, la 3-picoline, la 4-picoline, la quinoléine, la quinaldine et une alkylpyridine ; et
c) la mise en réaction du fluoroalkylnitrile neutralisé de formule I avec de l'azoture de sodium en présence d'un ou plusieurs solvant(s) approprié(s) afin d'obtenir le fluoroalkyltétrazole de formule II.

2. Procédé selon la revendication 1, dans lequel le sous-produit formé pendant ou après la réaction de déshydratation à partir de l'agent déshydratant est éliminé simultanément.

3. Procédé selon la revendication 1, dans lequel
a) l'étape (a) est réalisée à une température allant de -4 °C à 120 °C ;
b) le solvant utile à l'étape (a) est choisi dans le groupe constitué par l'acétonitrile, le tétrachlorure de carbone, le chloroforme, le dichlorométhane, l'acétone, la γ-butyrolactone, la N-méthyl-2-pyrrolidone, le nitrométhane, le diméthylformamide, la tétraméthylurée, la diméthylpropylène-urée, le diméthylsulfoxyde, le sulfolane, le carbonate de diméthyle, le carbonate d'éthylène, l'acide trifluoroacétique, l'acide difluoroacétique et l'acide acétique ;
c) le rapport molaire de l'agent déshydratant au fluoroalkylamide de formule III va de 0,5:1 à 20:1 ;
d) la concentration du fluoroalkylamide de formule III dans une solution contenant un ou plusieurs solvant(s) et le fluoroalkylamide de formule III va de 1,0 % p/p à 99,0 % p/p ; et
e) la concentration de l'agent déshydratant dans une solution contenant un ou plusieurs solvant(s) et l'agent déshydratant va de 1,0 % p/p à 99,0 % p/p.

4. Procédé selon la revendication 1, dans lequel
a) l'étape (b) est réalisée jusqu'à ce que le pH dans la plage allant de 6,5 à 8,5 soit atteint ; et
b) l'étape (b) est réalisée à une température allant de 15 °C à 60 °C.

5. Procédé selon la revendication 1, dans lequel
a) le solvant utilisé à l'étape (c) est choisi dans le groupe constitué par l'acétonitrile, le tétrachlorure de carbone, le chloroforme, le dichlorométhane, l'acétone, la γ-butyrolactone, la N-méthyl-2-pyrrolidone, le nitrométhane, le diméthylformamide, la tétraméthylurée, la diméthylpropylène-urée, le diméthylsulfoxyde, le sulfolane, le carbonate de diméthyle, le carbonate d'éthylène, l'acide trifluoroacétique, l'acide difluoroacétique et l'acide acétique ;
b) l'étape (c) est réalisée à une température allant de 0 °C à 120 °C ; et
c) le rapport molaire de l'azoture de sodium au fluoroalkylnitrile de formule I va de 1:1 à 1:10.
